# EUROPÄISCHE PATENTANMELDUNG

(11) **EP 4 681 648 A1**
(43) Veröffentlichungstag der Anmeldung: **21.01.2026**
(21) Anmeldenummer: 24189638.0
(22) Anmeldetag: 19.07.2024
(51) Int. Cl.: A61B 6/10

(54) **BEWEGUNGSSTEUERUNG EINER BEWEGBAREN EINHEIT EINER MEDIZINISCHEN ANLAGE**

(71) Anmelder: Siemens Healthineers AG, 91301 Forchheim (DE)
(72) Erfinder: GRIENER, Michael, 95478 Kemnath (DE); KARL, Harald, 90765 Fürth (DE); MEYNDT, Martin, 91080 Uttenreuth (DE); NOLEWAIKA, Martin, 91056 Erlangen (DE)
(74) Vertreter: Siemens Healthineers Patent Attorneys

(57) **Zusammenfassung**

Die Erfindung betrifft ein Verfahren zur Überwachung einer Bewegung wenigstens einer bewegbaren Einheit (2) einer medizinischen Anlage (1), wobei das Verfahren die folgenden Schritte umfasst:
(i) Generieren der Bewegung der Einheit (2) mittels wenigstens eines Aktors (6),
(ii) Generieren von Bewegungssignalen (25) anhand einer tatsächlich erfolgenden Bewegung der Einheit (2) und Übertragen der Bewegungssignale (25) an die Steuerungseinrichtung (9),
(iii) Überprüfen mittels der Steuerungseinrichtung (9),
(iv) Generieren von Fehlersignalen (29) mittels der Steuerungseinrichtung (9),
(v) Erfassen dreidimensionaler und die Einheit (2) betreffender Bilddaten (32) mittels einer Bilderfassungseinrichtung (33),
(vi) Generieren von Erwartungssignalen (36) mittels der Steuerungseinrichtung (9),
(vii) Überprüfen mittels der Überwachungseinrichtung (10), ob eine Abweichung vorhanden ist,
(viii) Generieren von Fehlersignalen (38) mittels der Überwachungseinrichtung (10).

## Beschreibung

Die vorliegende Erfindung betrifft ein Verfahren zur Überwachung einer Bewegung wenigstens einer bewegbaren Einheit einer medizinischen Anlage. Überdies betriff die vorliegende Erfindung eine entsprechende medizinische Anlage. Schließlich betrifft die vorliegende Erfindung ein Steuergerät für eine solche medizinische Anlage.

Bei medizinischen Anlagen, etwa Bildgebung- oder Behandlungseinrichtungen, erfolgt oft eine mechanische Bewegung einer Einheit der Anlage, etwa eines Patientenlagerungstischs oder einer Bildgebungseinheit. Grundsätzlich sind solche Bewegungen mit der Gefahr verbunden, dass Kollisionen stattfinden könnten, die zu einer Beschädigung der medizinischen Anlage oder gar einer Verletzung des Patienten führen könnten. Insofern werden an medizinische Anlagen hohe Anforderungen hinsichtlich einer diesbezüglichen Vermeidung gestellt, insbesondere die Realisierung einer Erstfehlersicherheit. So umfasst etwa die für medizinische, elektrische Geräte relevante europäische Norm IEC60601 diesbezüglich konkrete Vorgaben.

Die Erstfehlersicherheit wird typischerweise durch die Realisierung eines Signalpfades und eines unabhängig hiervon arbeitenden Sicherheitspfades, der auch als Kontrollpfad bezeichnet werden kann, bewerkstelligt. Der Signalpfad wird häufig auch als C-Pfad und der Sicherheitspfad als P-Pfad bezeichnet. Seitens des Signalpfades werden mittels einer Steuerungseinrichtung Steuersignale generiert, die die Bewegung der Einheit bewirken und an entsprechende Aktoren übertragen werden. Seitens des Sicherheitspfades werden erfasste Messdaten betreffend eine tatsächliche Bewegung der Einheit an eine Überwachungseinrichtung übersendet, mittels der im Falle einer Detektion eines diesbezüglichen Fehlers ein die Bewegung der Einheit unterbrechendes Signal generiert und ausgegeben werden. Die Erstfehlersicherheit wird hierbei dadurch realisiert, dass die in den jeweiligen Pfad eingebundenen Komponenten unabhängig voneinander arbeiten, sodass, sofern in der Sphäre von einem der Pfade ein Fehler auftritt, dennoch eine auf die Reduzierung der Kollisionsgefahr gerichtete Reaktion wie etwa das Anhalten der Bewegung erfolgt. Anders ausgedrückt pflanzt sich ein im Bereich eines der Pfade vorliegender Fehler nicht auf den jeweils anderen Pfad fort. Ein diesbezüglich einfaches Beispiel betrifft einen in den Sicherungspfad eingebundene Not-Aus-Schalter, dessen Betätigung zu einem unmittelbaren Anhalten der Einheit führt. Das mittels des Not-Aus-Schalters generierte Signal führt unmittelbar und unabhängig von den mittels der Steuerungseinrichtung generierten Steuersignalen zu einer Unterbrechung der jeweiligen Bewegung. Ein weiteres Konzept zur Realisierung einer Erstfehlersicherheit, bei dem als Messdaten die Daten von 3D-Kameras genutzt werden, ist aus US 2006 / 0 058 919 A1 und aus der bislang unveröffentlichten europäischen Patentanmeldung mit dem amtlichen Aktenzeichen EP 23200297.2 bekannt.

Ferner ist die Druckschrift US 2023 / 0 206 501 A1 zu nennen, in der die Verwendung einer 3D-Kamera bei einer medizinischen Anlage beschrieben ist. Konkret betrifft das dort beschriebene Konzept eine Kalibration betreffend Koordinatensysteme, die einer 3-D-Kamera und einer Bildgebungseinheit der medizinischen Anlage zugeordnet sind.

Die Erfindung stellt sich die Aufgabe, ein verbessertes Konzept hinsichtlich der Überwachung einer Bewegung einer Einheit einer medizinischen Anlage unter Verwendung dreidimensionaler Bilddaten anzugeben, insbesondere hinsichtlich der Realisierung einer Erstfehlersicherheit.

Erfindungsgemäß gelöst wird die Aufgabe bei einem Verfahren der eingangs genannten Art dadurch, dass dieses die folgenden Schritte umfasst:
(i) Generieren der Bewegung der Einheit mittels wenigstens eines Aktors dadurch, dass mittels einer Steuerungseinrichtung auf diese Bewegung gerichtete Steuersignale generiert und über eine Übertragungsleitung eines Signalpfades an den Aktor oder eine zur Ansteuerung des Aktors vorgesehene Komponente übertragen werden,
(ii) Generieren von Bewegungssignalen anhand einer tatsächlich erfolgenden Bewegung der Einheit mittels einer Bewegungserfassungseinrichtung und Übertragen der Bewegungssignale an die Steuerungseinrichtung über die oder eine weitere Übertragungsleitung des Signalpfades,
(iii) Überprüfen mittels der Steuerungseinrichtung, ob eine Abweichung zwischen einer tatsächlich erfolgenden Bewegung der Einheit, die anhand der Bewegungssignale bestimmt wird, und einer Bewegung der Einheit, die anhand der Steuersignale zu erwarten ist, vorhanden ist,
(iv) Generieren von Fehlersignalen mittels der Steuerungseinrichtung, falls die im Schritt (iii) erfolgende Überprüfung das Vorhandensein einer solchen Abweichung ergeben hat, wobei die Fehlersignale auf ein Reduzieren oder Beseitigen einer von der Bewegung der Einheit potentiell ausgehenden Gefahr gerichtet sind,
(v) Erfassen dreidimensionaler und die Einheit betreffender Bilddaten mittels einer Bilderfassungseinrichtung, wobei die Bilddaten über eine Übertragungsleitung eines Sicherheitspfades an eine Überwachungseinrichtung übertragen werden,
(vi) Generieren von Erwartungssignalen mittels der Steuerungseinrichtung, wobei die Erwartungssignale die zu generierende Bewegung der Einheit betreffen, und Übertragen der Erwartungssignale an die Überwachungseinrichtung,
(vii) Überprüfen mittels der Überwachungseinrichtung, ob eine Abweichung zwischen einer tatsächlich erfolgenden Bewegung der Einheit, die anhand der Bilddaten bestimmt wird, und einer Bewegung der Einheit, die anhand der Erwartungssignale zu erwarten ist, vorhanden ist,
(viii) Generieren von Fehlersignalen mittels der Überwachungseinrichtung, falls die im Schritt (vii) erfolgende Überprüfung das Vorhandensein einer solchen Abweichung ergeben hat, wobei die Fehlersignale auf ein Reduzieren oder Beseitigen einer von der Bewegung der Einheit potentiell ausgehenden Gefahr gerichtet sind.

Im Rahmen der Erfindung werden zwei parallele Verfahrensstränge unabhängig voneinander durchgeführt, wobei einer der Verfahrensstränge den Signalpfad und der andere der Verfahrensstränge den Sicherheitspfad realisiert. Während die Schritte (i) bis (iv) den Signalpfad betreffen, betreffend die Schritte (v) bis (viii) den Sicherheitspfad. Die diesbezüglich vorgesehenen Komponenten, also bezüglich des Steuerpfades die Steuerungseinrichtung und die Bewegungserfassungseinrichtung und bezüglich des Sicherheitspfades die Überwachungseinrichtung und die Bilderfassungseinrichtung, liegen als separate und unabhängig voneinander arbeitende Einheiten vor, sodass im Falle des Auftretens eines Fehlers bei einer dieser Einheiten nur der jeweilige Pfad, nicht jedoch der jeweils andere Pfad, betroffen ist. Die Durchführung der Schritte (i) bis (iv) und der Schritte (v) bis (viii) erfolgt bevorzugt parallel zueinander, also insbesondere zeitgleich.

Im ersten Schritt (i) werden mittels der Steuerungseinrichtung die die Bewegung initiierenden Steuersignale generiert und an den wenigstens einen Aktor oder an die zur Ansteuerung des Aktors vorgesehene Komponente ausgegeben. Die Übertragung der kann über einen Datenbus, etwa einem CAN-Bus, erfolgen. Die Generierung der Steuersignale kann anhand einer, insbesondere fest vorgegebenen, Durchführungsvorschrift wie etwa einem Ablaufplan oder Ablaufprotokoll erfolgen. Denkbar ist auch, dass die Generierung der Steuersignale anhand von Nutzersignalen, die seitens eines Nutzers vorgegeben werden, erfolgt. Diese Vorgabe kann mittels einer geeigneten Mensch-Maschine-Schnittstelle, etwa einem Joystick, erfolgen.

Im zweiten Schritt (ii) erfolgt die Generierung der Bewegungssignale, die eine tatsächliche, also physisch in der Realität vorliegende und messtechnisch erfasste, Bewegung der Einheit betrifft. So erfolgt in Abhängigkeit einer aktuellen Bewegung, also etwa einer aktuellen Lage und/oder Geschwindigkeit, die Generierung von Messsignalen, die die Bewegungssignale sind oder aus denen die Bewegungssignale bestimmt werden. Hierzu kann eine messtechnische Erfassung betreffend unmittelbar die Einheit selbst erfolgen. Die messtechnische Erfassung kann die Bewegung der Einheit auch mittelbar betreffen und beispielsweise über die Erfassung einer Bewegung erfolgen, die seitens des Aktors oder einer den Aktor mit der Einheit verbindenden Komponente vorliegt.

Im dritten Schritt (iii) wird mittels der Steuerungseinrichtung eine Überprüfung dahingehend vorgenommen, ob die gemessene Bewegung der Einheit und die auf die Bewegung der Einheit gerichteten Steuersignale miteinander in Einklang stehen. Sofern diese seitens des Signalpfades durchgeführte Überprüfung zu einer Übereinstimmung respektive zu keiner vorliegenden Abweichung führt, dann wird seitens des Signalpfades eine Fehlerfreiheit der Bewegung angenommen, sodass eine diesbezügliche Intervention, die ansonsten im Schritt (iv) durchgeführt werden würde, nicht erforderlich ist. Die Steuersignale werden in diesem Fall weiterhin derart generiert, dass die Bewegung der Einheit gemäß der Durchführungsvorschrift respektive der Nutzervorgabe weiter fortgesetzt wird. Andernfalls werden im Schritt (iv) als Interventionsmaßnahme die Fehlersignale generiert, die auf die Reduzierung oder Beseitigung einer potentiell von der Bewegung der Einheit ausgehenden Gefahr gerichtet sind, beziehungsweise diese bewirken. Konkrete Details diesbezüglich werden später erläutert.

Im fünften Schritt (v), der einen ersten Schritt des zur Realisierung des Sicherheitspfades vorgesehenen Verfahrensstrangs darstellt, werden die dreidimensionalen Bilddaten mittels der Bilderfassungseinrichtung erfasst. Die Bilderfassungseinrichtung kann wenigstens eine 3D-Kamera sein oder umfassen. Die Bilderfassungseinrichtung ist insbesondere zur Erfassung von Daten betreffend den visuell sichtbaren Teil des elektromagnetischen Spektrums eingerichtet. Zudem oder alternativ kann die Bilderfassungseinrichtung eine Einrichtung zur Erfassung von Infrarotlicht und/oder eine Lidareinrichtung sein oder umfassen. Die dreidimensionalen Bilddaten bilden die Einheit samt ihrer Umgebung ab. Die Bilderfassungseinrichtung respektive die 3D-Kamera kann oberseitig an der medizinischen Anlage angeordnet sein, wobei das entsprechende Blickfeld nach unten gerichtet ist. Bevorzugt umfasst die Bilderfassungseinrichtung mehrere 3D-Kameras mit unterschiedlichen Blickfeldern, wobei der komplette Bereich der medizinischen Anlage von den Blickfeldern erfasst wird und alle denkbaren Positionen der Einheit in zumindest einem der Blickfelder enthalten ist.

Die Bilddaten können als ein zweidimensionales Array an Pixeln vorliegen, wobei jedem Pixel ein Tiefenwert zugeordnet ist, der einen Abstand der dort abgebildeten Komponente zur Bilderfassungseinrichtung betrifft. Denkbar ist auch, dass die Bilddaten als ein dreidimensionales Array an Voxeln vorliegen. Zur Verarbeitung und Auswertung der Bilddaten ist die Überwachungseinrichtung ausgebildet und eingerichtet. So kann auf der Überwachungseinrichtung eine hierfür geeignete Bildauswertungssoftware implementiert sein. Insbesondere erfolgt im Rahmen der Auswertung der Bilddaten eine Segmentierung der Bilddaten, bei der in den Bilddaten vorhandene Bereiche der Einheit oder anderen sich im Bereich der medizinischen Anlage befindenden Komponenten zugeordnet werden. In jedem Fall erfolgt die Übertragung der Bilddaten zur Überwachungseinrichtung über eine Übertragungsleitung, die eine bezüglich der Übertragung der Steuersignale von der Steuerungseinrichtung zu dem Aktor oder der zur Ansteuerung des Aktors vorgesehenen Komponente sowie der Bewegungssignale von der Bewegungserfassungseinrichtung zur Steuerungseinrichtung andere Übertragungsleitung ist.

Im sechsten Schritt (vi), der einen zweiten Schritt des zur Realisierung des Sicherheitspfades vorgesehenen Verfahrensstrangs darstellt, werden mittels der Steuerungseinrichtung die Erwartungssignale generiert und an die Überwachungseinrichtung übertragen. Diese Übertragung erfolgt bevorzugt über eine bezüglich der übrigen Übertragungsleitungen separate Übertragungsleitung. Bevorzugt sind die Erwartungssignale nicht die Steuersignale selbst, sondern werden, gleichermaßen die Steuersignale, anhand der Durchführungsvorschrift respektive der Nutzervorgabe erzeugt, sodass die Erwartungssignale nicht von einem etwaigen Fehler, der bei der Erzeugung der Steuersignale auftritt, beeinflusst werden.

Im siebten Schritt (vii), der einen dritten Schritt des zur Realisierung des Sicherheitspfades vorgesehenen Verfahrensstrangs darstellt, wird mittels der Überwachungseinrichtung eine Überprüfung dahingehend vorgenommen, ob die anhand der Bilddaten ermittelte Bewegung der Einheit und die auf die zu erwartende Bewegung der Einheit gerichteten Erwartungssignale miteinander in Einklang stehen. Sofern diese seitens des Sicherheitspfades durchgeführte Überprüfung zu einer Übereinstimmung diesbezüglich respektive zu keiner vorliegenden Abweichung führt, dann wird seitens des Sicherheitspfades von einer Fehlerfreiheit der Bewegung ausgegangen, sodass eine entsprechende Intervention, die ansonsten im Schritt (viii) durchgeführt werden würde, nicht erforderlich ist. Die Fehlersignale werden in diesem Fall nicht generiert, sodass die Bewegung der Einheit weiterhin fortgesetzt werden kann. Andernfalls werden im Schritt (viii), der einen vierten Schritt des zur Realisierung des Sicherheitspfades vorgesehenen Verfahrensstrangs darstellt, als entsprechende Interventionsmaßnahme die Fehlersignale mittels der Überwachungseinrichtung generiert, die, allgemein gesprochen, auf die Reduzierung oder Beseitigung einer potentiell von der Bewegung der Einheit ausgehenden Gefahr gerichtet sind beziehungsweise diese bewirken. Auch konkrete Details diesbezüglich werden später erläutert.

In den Schritten (iii) und (vii) erfolgt eine Überprüfung zweier Vergleichsgrößen, wobei nur bei deren Übereinstimmung von einer fehlerfreien Durchführung der Bewegung der Einheit im Rahmen des jeweiligen Pfades ausgegangen wird. Während im dritten Schritt (iii) diesbezüglich die Vergleichsgrößen anhand der Bewegungssignale einerseits und anhand der Steuersignale andererseits generiert werden, werden im siebten Schritt (vii) die Vergleichsgrößen anhand der Bilddaten einerseits und anhand der Erwartungssignale andererseits generiert.

Als die Vergleichsgrößen können Bewegungsinformationen ermittelt werden, die eine Position und/oder eine Geschwindigkeit und/oder eine Beschleunigung der Einheit betreffen oder beschreiben. Hierbei kann ein bezüglich der medizinischen Anlage, der Bilderfassungseinrichtung oder der Einheit ortsfestes Bezugssystem verwendet werden. Hierbei können auch mehrere solcher Bezugssysteme Verwendung finden, wobei zur Ermöglichung einer Vergleichbarkeit Koordinatentransformationen durchgeführt werden können.

Bezüglich des dritten Schritts (iii) können als die Vergleichsgrößen eine erste Bewegungsinformation und eine zweite Bewegungsinformation ermittelt werden. Die erste Bewegungsinformation wird anhand der Steuersignale ermittelt und betrifft die Bewegung der Einheit, wie sie bei einem fehlerfreien Betrieb anhand der Steuersignale zu erwarten wäre. Die zweite Bewegungsinformation wird anhand der Bewegungssignale ermittelt und betrifft die tatsächliche respektive in Realität vorliegende Bewegung der Einheit.

Bezüglich des siebten Schritts (vii) können als die Vergleichsgrößen eine dritte Bewegungsinformation und eine vierte Bewegungsinformation ermittelt werden. Die dritte Bewegungsinformation wird anhand der Bilddaten ermittelt und betrifft die tatsächliche respektive in Realität vorliegende Bewegung der Einheit. Die dritte Bewegungsinformation kann dadurch ermittelt werden, dass die Bilddaten bezüglich wenigstens eines Referenzpunkts der Einheit, insbesondere unter zusätzlicher Verwendung bekannter Abmessungen und Geometrien der Einheit, ausgewertet werden. Als Referenzpunkte können an der Einheit angebrachte Markierungen oder markante Stellen der Einheit vorgesehen sein. Die vierte Bewegungsinformation wird anhand der Erwartungssignale ermittelt und betrifft die Bewegung der Einheit, wie sie bei einem fehlerfreien Betrieb anhand der Steuersignale zu erwarten wäre.

Einer der zentralen Vorteile der vorliegenden Erfindung betrifft die denkbar einfache Realisierbarkeit des Sicherheitspfades. So wird diesbezüglich als das Erfassungsmittel für die Messdaten eine ohnehin häufig vorgesehene Komponente der medizinischen Anlage genutzt, nämlich die Bilderfassungseinrichtung. So finden 3D-Kameras bereits aktuell häufig Verwendung, beispielsweise im Zuge der Durchführung einer Bildgebung respektive Behandlung, etwa für Zwecke einer Vermessung oder Positionsbestimmung des Patienten. Im Stand der Technik ist nicht selten eine Kodiereinrichtung zur Realisierung des Signalpfades vorgesehen, wobei eine weitere Kodiereinrichtung zur Realisierung des Sicherheitspfades vorgesehen ist. Die Notwendigkeit der weiteren Kodiereinrichtung entfällt bei der vorliegenden Erfindung, sodass auch das Erfordernis einer entsprechenden Verkabelung wegfällt.

Denkbar ist, dass wenigstens ein einen Frequenzumrichter realisierender Motor-Controller vorgesehen ist, mittels dem der hiermit verbundene elektromechanische Aktor mit einer zum Generieren der Bewegung der Einheit erforderlichen Betriebsspannung beaufschlagbar ist, wobei die in Schritt (i) generierten Steuersignale zur Steuerung des Aktors zum Motor-Controller übertragen werden. Grundsätzlich kann der Aktor ein, insbesondere dreiphasiger, Drehstrommotor sein. Der Motor-Controller verbindet eine Spannungsquelle mit dem Aktor, wobei anhand der Steuersignale eine Amplitude und/oder eine Frequenz der an den Aktor anliegenden Spannung gesteuert wird. Der Motor-Controller weist zu diesem Zweck eine Schnittstelle auf, die mit der Übertragungsleitung, über die die Steuersignale übertragen werden, verbunden ist. Alternativ kann der Aktor auch als ein Gleichstrommotor oder ein Schrittmotor ausgebildet sein.

Bei dem erfindungsgemäßen Verfahren kann vorgesehen sein, dass die Bewegungserfassungseinrichtung eine Kodiereinrichtung ist, mittels der die tatsächlich erfolgende Bewegung der Einheit und/oder des Aktors im Rahmen des Schritts (ii) in die Bewegungssignale umgewandelt wird. Die Kodiereinrichtung kann auch als ein Encoder bezeichnet werden. Mittels der Kodiereinrichtung werden anhand der aktuellen Stellung der Einheit respektive einer hiermit verbundenen Komponente die Bewegungssignale respektive Messsignale, aus denen die Bewegungssignale ermittelt werden, generiert. So kann diesbezüglich die aktuelle Stellung einer Schubstange bezüglich einer linearen Bewegung oder eines Gelenks bezüglich seiner Schwenkstellung ermittelt werden.

Bevorzugt ist erfindungsgemäß vorgesehen, dass die Bewegung der Einheit mittels mehrerer Aktoren durch die Generierung von Steuersignalen, die an die Aktoren oder die zur Ansteuerung der Aktoren vorgesehenen Komponenten übertragen werden, generiert wird, sodass die Bewegung eine Gesamtbewegung ist, die sich aus Einzelbewegungen, die mittels der Aktoren jeweils generiert werden, zusammensetzt. Im Rahmen dieser Ausführungsform kann eine sogenannte Multi-Achs-Bewegung realisiert werden, wobei die einzelnen Bewegungen jeweils lineare Bewegungen oder Schwenkbewegungen sein können, die sich in Summe zu der Gesamtbewegung zusammensetzen und beispielsweise die Bewegung der Einheit entlang einer gekrümmten Trajektorie ermöglichen. Im Rahmen dieser Ausführungsform wird in Schritt (vii) überprüft, ob eine Abweichung zwischen der tatsächlich erfolgenden Gesamtbewegung der Einheit, die anhand der Bilddaten bestimmt wird, und der Gesamtbewegung der Einheit, die anhand der Erwartungssignale zu erwarten ist, vorhanden ist.

Besonders bevorzugt bewirken die im Rahmen des Schritts (iv) oder des Schritts (viii) generierten Fehlersignale ein Anhalten der Bewegung der Einheit. So ist das Anhalten dieser Bewegung, also die Reduzierung der Geschwindigkeit der Einheit auf Null, eine effektive Maßnahme, um eine etwaige, aufgrund der Bewegung vorliegende Gefahr zu eliminieren. Konkret können die im Schritt (iv) generierten Fehlersignale seitens der Steuerungseinrichtung generierte Steuersignale sein, die ein Anhalten der Bewegung der Einheit bewirken. Es erfolgt mithin ein aktiver Eingriff in die Steuerung mittels der Steuerungseinrichtung. Denkbar ist auch, dass die im Schritt (viii) generierten Fehlersignale seitens der Überwachungseinrichtung generiert und an eine Unterbrechungseinrichtung und/oder eine Bremseinrichtung ausgegeben werden und eine mittels der Unterbrechungseinrichtung bewirkte Unterbrechung einer Beaufschlagung des Aktors mit der oder einer Betriebsspannung und/oder ein Festsetzen der Einheit mittels der Bremseinrichtung bewirken. Die Unterbrechungseinrichtung ist bevorzugt ein Schalter, mittels dem die Stromzufuhr zu dem Aktor unterbrochen werden kann. Insbesondere um eine aufgrund der Massenträgheit und/oder der Schwerkraft weiterhin auftretende Bewegung der Einheit zu vermeiden, bewirkt die Bremseinrichtung ein Feststellen der Einheit. Die Bremseinrichtung kann zu diesem Zweck Bremsscheiben und/oder -backen aufweisen, die mit einem sich während der Bewegung der Einheit bewegenden Element entsprechend vermittels Reibkräften zusammenwirkt.

Denkbar ist, dass die im Rahmen des Schritts (iv) oder des Schritts (viii) generierten Fehlersignale an eine Ausgabeeinrichtung übertragen werden und die mittels der Ausgabeeinrichtung erfolgende Ausgabe einer Fehlermitteilung bewirken, mittels der ein Nutzer auf das Vorliegen einer potentiellen Störsituation hingewiesen wird. Die Ausgabeeinrichtung kann zur Ausgabe einer optischen und/oder akustischen Fehlermitteilung eingerichtet sein. So kann die Ausgabeeinrichtung ein Display respektive Touchscreen und/oder einen Lautsprecher umfassen. Die Fehlermitteilung kann als ein Text ausgegeben werden. So kann der Nutzer etwa über den Text "Achtung, potentielle Störung oder Kollisionsgefahr bei der Bewegung" entsprechend informiert werden, sodass auch dieser gegebenenfalls Gegenmaßnahmen wie etwa die Betätigung eines, beispielsweise in einen weiteren Sicherheitspfad eingebundenen, Not-Aus-Schalters vornehmen kann.

Sofern die jeweiligen Fehlersignale sowohl das Anhalten der Bewegung der Einheit als auch die Ausgabe der Fehlermitteilung bewirken, dann kann nach erfolgter Generierung der das Anhalten der Bewegung der Einheit und die Ausgabe der Fehlermitteilung bewirkenden Fehlersignale zusätzlich die Erfüllung einer Freigabebedingung überprüft werden, wobei die Freigabebedingung erfüllt ist, wenn ein mittels einer Eingabevorrichtung generiertes Freigabesignal vorliegt, wobei die Generierung des Freigabesignals mittels einer nutzerseitig an der Eingabevorrichtung vorgenommenen und auf die Freigabe der Bewegung gerichteten Nutzerhandlung erfolgt, wobei die Bewegung der Einheit nur bei der Erfüllung der Freigabebedingung, insbesondere im Rahmen einer reduzierten Geschwindigkeit, fortgesetzt wird. Diese Ausführungsform betrifft den Fall, bei dem der Nutzer, also bezüglich der Bedienung der medizinischen Anlage geschultes Fachpersonal, bei einem seitens des Signalpfades und/oder seitens des Sicherheitspfades detektierten Fehler dennoch die Fortführung der jeweiligen Bewegung wünscht. Dies kann der Fall sein, wenn der Nutzer erkennt, dass trotz des vorliegenden Fehlers keine Kollisionsgefahr von der sich bewegenden Einheit ausgeht.

Bevorzugt ist vorgesehen, dass die Bilddaten zur Steuerungseinrichtung übertragen werden, wobei die Steuersignale, insbesondere zusätzlich, anhand der Bilddaten generiert werden. Hierbei werden die Bilddaten synergetisch nicht nur zur Realisierung des Sicherheitspfades genutzt, sondern einem weiteren Zweck zugeführt, nämlich der Generierung der Steuersignale.

So können die Bilddaten seitens der Steuerungseinrichtung dahingehend ausgewertet werden, dass diesbezüglich eine bildbasierte Bewegungsinformation betreffend die Einheit erfasst wird, wobei die bildbasierte Bewegungsinformation mit einer Bewegungsinformation verglichen wird, die anhand der Bewegungssignale ermittelt wurde und die bereits weiter oben als zweite Bewegungsinformation bezeichnet wurde. Sofern ein Abgleich dieser beiden Bewegungsinformationen erfolgt, kann, sofern die ermittelte Abweichung kleiner als ein vorgegebener Grenzwert ist, davon ausgegangen werden, dass aktuell kein Fehler vorliegt und die Steuersignale bezüglich der Bewegung der Einheit weiterhin generiert und ausgegeben werden können. Hierdurch wird eine weitere, seitens des Signalpfades vorliegende Sicherheitsebene realisiert. Grundsätzlich kann diesbezüglich eine Bewegungssteuerung der beweglichen Einheit gemäß dem in der bereits eingangs erwähnten europäischen Patentanmeldung EP 23200297.2 erfolgen, auf die entsprechend Bezug genommen wird.

Konkret ist auch denkbar, dass mittels der Steuerungseinrichtung und anhand der Bilddaten wenigstens eine eine aktuelle Position und/oder Bewegung der Einheit betreffende Einheitinformation und eine Objektinformation betreffend eine aktuelle Position und/oder Bewegung eines sich im Bereich der medizinischen Anlage befindenden Objekts ermittelt wird, wobei die Steuersignale anhand der wenigstens einen Einheitinformation und/oder der wenigstens einen Objektinformation generiert werden. Bei dem Objekt kann es sich um jedweden Gegenstand, einschließlich dem Patienten, und/oder jedwede Einheit der medizinischen Anlage handeln, der und/oder die sich im Bereich der medizinischen Anlage befindet. Das Objekt kann bezüglich der medizinischen Anlage ortsfest sein. Bei dem Objekt kann es sich jedoch auch um eine weitere bewegbare Einheit der medizinischen Einrichtung handeln. Die entsprechende Datenauswertung erfolgt insbesondere anhand der bereits weiter oben genannten Segmentierung der Bilddaten.

Denkbar ist, dass mittels der Steuerungseinrichtung die Erfüllung einer Kollisionsbedingung überprüft wird, die dann erfüllt ist, wenn sich anhand der wenigstens einen Einheitinformation und der wenigstens einen Objektinformation ergibt, dass aktuell die Gefahr einer Kollision der Einheit und des Objekts gegeben ist, wobei mittels der Steuerungseinrichtung bei der Erfüllung der Kollisionsbedingung die Steuersignale derart generiert werden, dass die Gefahr verringert oder beseitigt wird. So können anhand der Einheitinformation und/oder der Objektinformation voraussichtliche Trajektorien der Einheit und/oder des Objekts ermittelt werden, wobei die Kollisionsbedingung dann erfüllt ist, wenn diese Trajektorien einen Schnittpunkt oder, allgemein gesprochen, einen gemeinsamen Bereich aufweisen. Insbesondere wenn Abmessungen und Geometrien der Einheit und/oder des Objekts bekannt sind, dann können als die Trajektorien die Volumenbereiche des Bereichs der medizinischen Anlage bestimmt werden, die von der Einheit respektive dem Objekt überstrichen werden. Die Kollisionsbedingung kann dann erfüllt sein, wenn diese Volumenbereiche nicht disjunkt sind. Zusätzlich kann zur Erfüllung der Kollisionsbedingung eine zeitliche Bedingung überprüft werden, die dann erfüllt ist, wenn sich anhand der voraussichtlichen Trajektorie(n) ergibt, dass sich die Einheit und das Objekt zum selben Zeitpunkt voraussichtlich an derselben Position befinden.

Weiterhin betrifft die vorliegende Erfindung eine medizinische Anlage, umfassend wenigstens eine bewegbare Einheit und eine Steuerungseinrichtung, die dazu eingerichtet ist, auf die Bewegung der Einheit gerichtete und diese Bewegung bewirkende Steuersignale zu generieren und über eine Übertragungsleitung eines Signalpfades an einen Aktor zu übertragen, und eine Bewegungserfassungseinrichtung, mittels der anhand einer tatsächlich erfolgenden Bewegung der Einheit Bewegungssignale generierbar und an die Steuerungseinrichtung über die oder eine weitere Übertragungsleitung des Signalpfades übertragbar sind, wobei die Steuerungseinrichtung ferner dazu eingerichtet ist, zu überprüfen, ob eine Abweichung zwischen einer tatsächlich erfolgenden Bewegung der Einheit, die anhand der Bewegungssignale bestimmbar ist, und einer Bewegung der Einheit, die anhand der Steuersignale zu erwarten ist, vorhanden ist, und im Falle des Vorhandenseins einer solchen Abweichung Fehlersignale zu generieren, wobei die Fehlersignale auf ein Reduzieren oder Beseitigen einer von der Bewegung der bewegbaren Einheit potentiell ausgehenden Gefahr gerichtet sind, wobei die medizinische Anlage weiterhin eine Bilderfassungseinrichtung umfasst, mittels der dreidimensionale und die Einheit betreffende Bilddaten erfassbar und über eine Übertragungsleitung eines Sicherheitspfades an eine Überwachungseinrichtung der medizinischen Anlage übertragbar sind, wobei die Steuerungseinrichtung ferner dazu eingerichtet ist, Erwartungssignale, die die zu generierende Bewegung der Einheit betreffen zu generieren und an die Überwachungseinrichtung auszugeben, wobei die Überwachungseinrichtung dazu eingerichtet ist, zu überprüfen, ob eine Abweichung zwischen einer tatsächlich erfolgenden Bewegung der Einheit, die anhand der Bilddaten bestimmbar ist, und einer Bewegung der Einheit, die anhand der Erwartungssignale zu erwarten ist, vorhanden ist, und im Falle des Vorhandenseins einer solchen Abweichung Fehlersignale zu generieren, wobei die Fehlersignale auf ein Reduzieren oder Beseitigen einer von der Bewegung der Einheit potentiell ausgehenden Gefahr gerichtet sind. Alle im Zusammenhang mit dem erfindungsgemäßen Verfahren erläuterten Merkmale, Vorteile und Aspekte sind gleichermaßen auf die erfindungsgemäße medizinische Anlage übertragbar und umgekehrt.

Die erfindungsgemäße medizinische Anlage kann eine medizinische Bildgebungseinrichtung sein. Bei der medizinischen Bildgebungseinrichtung kann es sich um eine Magnetresonanztomographie-Einrichtung oder eine Computertomographie-Einrichtung oder ein Angiographiegerät oder ein Ultraschallgerät oder eine Positronen-Emissions-Tomographie-Anlage handeln. Denkbar ist auch, dass die erfindungsgemäße medizinische Anlage eine Behandlungseinrichtung, insbesondere eine Strahlentherapie-Anlage, ist.

Die Einheit oder eine der Einheiten kann ein Patientenlagerungstisch zur Aufnahme eines Patienten zur Durchführung einer Bildgebung oder Behandlung oder eine Bildgebungseinheit, insbesondere ein C-Bogen oder eine Vorrichtung zur Erzeugung einer ionisierenden Strahlung, sein. Insbesondere wird die Einheit von einer Umpositionierungsvorrichtung, mittels der die Bewegung der Einheit generierbar ist und die insbesondere den Aktor umfasst, getragen. Bei der Umpositionierungsvorrichtung kann es sich um einen mittels dem wenigstens einen Aktor beweglichen Knick- beziehungsweise Tragarm handeln.

Überdies betrifft die vorliegende Erfindung ein Steuergerät für eine medizinische Anlage gemäß der vorangehenden Beschreibungspassage, wobei das Steuergerät als eine Steuerungseinrichtung oder eine Überwachungseinrichtung, ausgebildet ist, die zur Durchführung des Verfahrens gemäß obiger Beschreibung eingerichtet ist. Auf dem Steuergerät kann ein Computerprogramm implementiert sein, mittels dem zumindest ein Teil der Schritte des erfindungsgemäßen Verfahrens, insbesondere automatisiert, durchführbar sind. Die Ausführung des Computerprogramms bewirkt mithin zumindest eine Ausführung eines Teiles der oben beschriebenen Schritte (i) bis (vii). Alle im Zusammenhang mit dem erfindungsgemäßen Verfahren und der erfindungsgemäßen medizinischen Anlage erläuterten Vorteile, Merkmale und Aspekte sind gleichermaßen auf das erfindungsgemäße Steuergerät übertragbar und umgekehrt.

Ferner betrifft die vorliegende Erfindung ein Computerprogramm, welches in eine Speichereinheit eines Steuergeräts gemäß der vorangehender Beschreibungspassage ladbar ist, umfassend Programmabschnitte, um Schritte des Verfahrens nach obiger Beschreibung auszuführen, wenn das Computerprogramm seitens des Steuergeräts ausgeführt wird. Alle im Zusammenhang mit dem erfindungsgemäßen Verfahren, der erfindungsgemäßen medizinischen Anlage und dem erfindungsgemäßen Steuergerät erläuterten Vorteile, Merkmale und Aspekte sind gleichermaßen auf das erfindungsgemäße Computerprogramm übertragbar und umgekehrt.

Schließlich betrifft die vorliegende Erfindung ein computerlesbares Medium, auf welchem von einem Steuergerät gemäß obiger Beschreibung einlesbare und ausführbare Programmabschnitte gespeichert sind, um Schritte des Verfahrens nach obiger Beschreibung auszuführen, wenn die Programmabschnitte seitens des Steuergeräts ausgeführt werden. Alle im Zusammenhang mit dem erfindungsgemäßen Verfahren, der erfindungsgemäßen medizinischen Anlage, dem erfindungsgemäßen Steuergerät und dem erfindungsgemäßen Computerprogramm erläuterten Vorteile, Merkmale und Aspekte sind gleichermaßen auf das erfindungsgemäße computerlesbare Medium übertragbar und umgekehrt.

Weitere Vorteile und Einzelheiten der vorliegenden Erfindung ergeben sich aus den nachfolgend dargelegten Ausführungsbeispielen sowie anhand der Figuren. Diese zeigen schematisch:
- Fig. 1:: eine Prinzipskizze einer erfindungsgemäßen medizinischen Anlage gemäß einem Ausführungsbeispiel, umfassend ein als eine Steuerungseinrichtung ausgebildetes erfindungsgemäßes Steuergerät gemäß einem Ausführungsbeispiel und ein als eine Überwachungseinrichtung ausgebildetes erfindungsgemäßes Steuergerät gemäß einem Ausführungsbeispiel,
- Fig. 2:: eine schematische Darstellung der medizinischen Anlage der Fig. 1,
- Fig. 3:: ein Flussdiagramm zur Veranschaulichung eines erfindungsgemäßen Verfahrens gemäß einem Ausführungsbeispiel, das anhand der in der Fig. 1 gezeigten medizinischen Anlage durchgeführt wird, und
- Fig. 4:: eine detaillierte Ansicht des Flussdiagramms der Fig. 3 betreffend den ersten Schritt dieses Verfahrens.

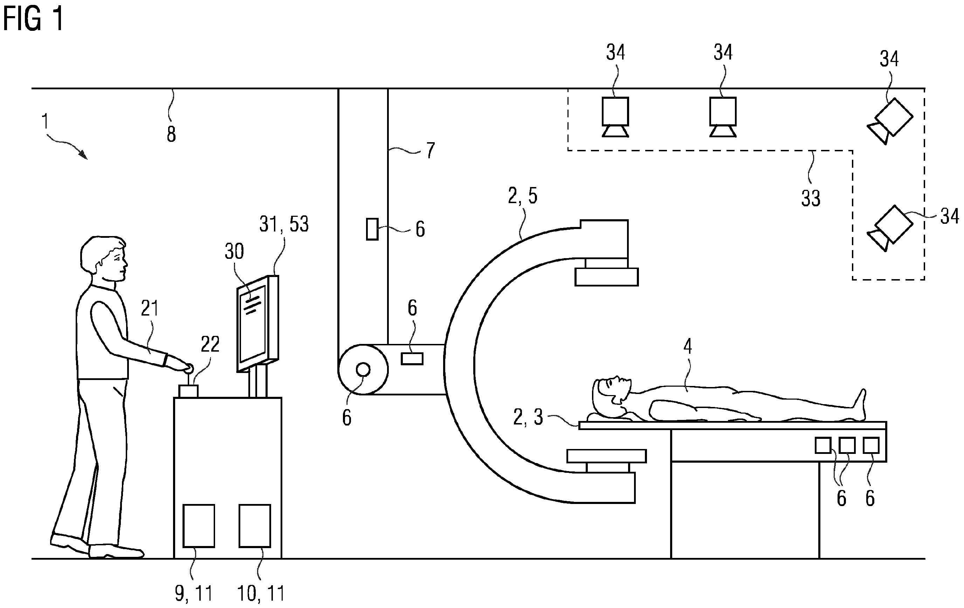

Fig. 1 zeigt ein Ausführungsbeispiel einer medizinischen Anlage 1, die vorliegend eine medizinische Bildgebungseinrichtung ist. Alternativ kann die medizinische Anlage 1 eine Behandlungseinrichtung sein. Wesentlich für die vorliegende Erfindung ist lediglich, dass die medizinische Anlage 1 wenigstens eine bewegbare Einheit 2 umfasst, die im Rahmen der Durchführung einer Bildgebung respektive Behandlung umpositionierbar beziehungsweise verfahrbar ist.

Bei der medizinischen Anlage 1 gemäß dem vorliegenden Ausführungsbeispiel sind exemplarisch zwei solche Einheiten 2 vorgesehen, nämlich ein Patientenlagerungstisch 3 zur Aufnahme eines Patienten 4 während der Durchführung der Bildgebung sowie eine als ein C-Bogen ausgebildete Bildgebungseinheit 5. Der Patientenlagerungstisch 3 ist mit drei Aktoren 6 gekoppelt, mittels denen jeweils eine Bewegung des Patientenlagerungstisches 3 vornehmbar ist, nämlich bezüglich der Hoch-Tief-, der Links-Rechts- und der Vor-Zurück-Richtung. Die Bildgebungseinheit 5 wird von einer Umpositionierungsvorrichtung, die ein an der Decke 8 montierter Tragarm 7 ist, getragen. Der Tragarm 7 umfasst ebenfalls Aktoren 6, mittels denen translatorische Bewegungen sowie Schwenkbewegungen der Bildgebungseinheit 5 durchführbar sind.

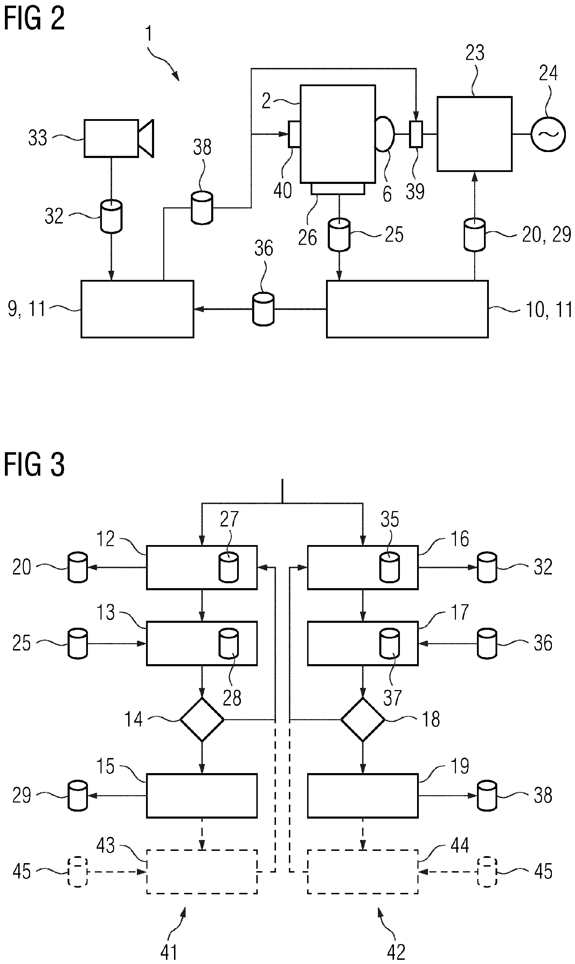

Fig. 2 zeigt eine äußerst schematische Darstellung der medizinischen Anlage 1, wobei übertragene Signale durch Zylinder symbolhaft angedeutet sind. Fig. 3 zeigt ein Flussdiagramm zur Veranschaulichung des erfindungsgemäßen Verfahrens gemäß einem Ausführungsbeispiel, das nachfolgend anhand der in den Figuren 1 und 2 gezeigten medizinischen Anlage 1 erläutert wird. Zur Durchführung des erfindungsgemäßen Verfahrens umfasst die medizinische Anlage 1 eine Steuerungseinrichtung 9 sowie eine Überwachungseinrichtung 10, die jeweils ein erfindungsgemäßes Steuergerät 11 gemäß jeweils einem Ausführungsbeispiel realisieren. Vorgesehen ist für jedes der Steuergeräte 11 jeweils ein erfindungsgemäßes Computerprogramm gemäß einem Ausführungsbeispiel, das in eine Speichereinheit des jeweiligen Steuergerätes 11 ladbar ist. Jedes der Computerprogramme umfasst Programmabschnitte, um Schritte des Verfahrens auszuführen, wenn das Computerprogramm seitens des jeweiligen Steuergeräts 11 ausgeführt wird. Weiterhin umfassen die Steuergeräte 11 jeweils ein erfindungsgemäßes, computerlesbares Medium gemäß jeweils einem Ausführungsbeispiel, auf denen jeweils von dem jeweiligen Steuergerät 11 einlesbare und ausführbare Programmabschnitte gespeichert sind, um Schritte des Verfahrens nach obiger Beschreibung auszuführen, wenn die Programmabschnitte seitens des jeweiligen Steuergeräts 11 ausgeführt werden. Das Verfahren umfasst die Schritte 12 bis 19, wobei die Schritte 12 bis 15 und 16 bis 19, wie in der Fig. 3 auch entsprechend angedeutet ist, jeweils parallel und zeitgleich zueinander durchgeführt werden. Die Schritte 12 bis 15 realisieren hierbei einen Signalpfad 41 und die Schritte 16 bis 19 einen Sicherheitspfad 42.

Im ersten Schritt 12 des zur Realisierung des Signalpfades 41 vorgesehenen Verfahrensstrangs erfolgt mittels der Steuerungseinrichtung 9 die Generierung von Steuersignalen 20, mittels denen die Aktoren 6 jeweils zur Generierung einer gewünschten Bewegung der jeweiligen Einheit 2 angesteuert werden. Die Übertragung der Steuersignale 20 an den jeweiligen Aktor 6 erfolgt über einen Datenbus, nämlich einem CAN-Bus. Die Generierung der Steuersignale 20 erfolgt entweder anhand einer fest vorgegebenen Durchführungsvorschrift wie beispielsweise einem entsprechenden Ablaufplan, der seitens der Steuerungseinrichtung 9 hinterlegt respektive abgespeichert ist. Alternativ erfolgt die Generierung der Steuersignale 20 anhand von seitens eines Nutzers 21 generierten Nutzersignalen, die dieser mittels einer Mensch-Maschine-Schnittstelle 22, die vorliegend ein Joystick ist, generiert werden.

Anhand der Steuersignale 20 wird eine erste Bewegungsinformation 27 ermittelt, die die Bewegung der jeweiligen Einheit 2 betrifft beziehungsweise beschreibt, wie sie bei einem fehlerfreien Betrieb anhand der Steuersignale 20 zu erwarten wäre. Konkret betrifft die erste Bewegungsinformation 27 die entsprechend aktuelle Position sowie die Geschwindigkeit der jeweiligen Einheit 2. Die erste Bewegungsinformation 27 umfasst die Koordinaten der jeweiligen Einheit 2 sowie deren Geschwindigkeit hinsichtlich Betrag und Richtung.

Insbesondere unter Bezugnahme auf Fig. 2 wird ersichtlich, dass die Steuerung der Aktoren 6 mittels der Steuersignale 20 mittelbar erfolgt. Der Übersichtlichkeit halber zeigt Fig. 2 nur einen der Aktoren 6, wobei für die anderen Aktoren 6 das entsprechend Erläuterte gleichermaßen gilt. So ist für jeden der Aktoren 6 ein einen Frequenzumrichter realisierender Motor-Controller 23 vorgesehen, mittels dem der hiermit verbundene elektromechanische Aktor 6 mit einer zum Generieren der Bewegung der mit dem jeweiligen Aktor 6 gekoppelten Einheit 2 erforderlichen Betriebsspannung beaufschlagbar ist. Die Steuersignale 20 werden mithin an den Motor-Controller 23 übertragen. Die Aktoren 6 sind jeweils als ein dreiphasiger Drehstrommotor ausgebildet. Der Motor-Controller 23 verbindet eine Spannungsquelle 24 mit dem jeweiligen Aktor 6, wobei anhand der Steuersignale 20 eine Amplitude sowie eine Frequenz der an den jeweiligen Aktor 6 anliegenden Spannung gesteuert wird. Denkbar ist auch, dass der Aktor 6 ein Gleichstrommotor oder ein Schrittmotor ist.

Im zweiten Schritt 13 des zur Realisierung des Signalpfades 41 vorgesehenen Verfahrensstrangs erfolgt eine Generierung von Bewegungssignalen 25 mittels einer Bewegungserfassungseinrichtung 26. Die Übertragung der Bewegungssignale 25 zur Steuerungseinrichtung 9 erfolgt, gleichermaßen wie die Übertragung der Steuersignale 20 zum Aktor 6 respektive zum Motor-Controller 23, über eine entsprechende Übertragungsleitung des Signalpfades 41. Die Bewegungserfassungseinrichtung 26 ist eine Kodiereinrichtung respektive ein Encoder, mittels der die tatsächlich erfolgende Bewegung der Einheit 2 respektive des mit der Einheit 2 verbundenen Aktors 6 messtechnisch erfassbar ist. Mittels der Bewegungserfassungseinrichtung 26 werden anhand der aktuellen Stellung der Einheit 2 respektive einer hiermit verbundenen Komponente des Aktors 6 die Messsignale generiert, aus denen die Bewegungssignale 25 ermittelt werden oder die Bewegungssignale 25 sind. Je nachdem, um welchen der Aktoren 6 es sich hierbei vorliegend konkret handelt, wird diesbezüglich die aktuelle Stellung bezüglich der translatorisch-linearen Bewegung einer Schubstange oder der Schwenkbewegung eines Gelenks ermittelt.

Mittels der Steuerungseinrichtung 9 wird anhand der Bewegungssignale 25 eine zweite Bewegungsinformation 28 ermittelt. Die zweite Bewegungsinformation 28 betrifft die tatsächliche respektive in Realität vorliegende Bewegung der Einheit 2. Die zweite Bewegungsinformation 28 umfasst die Koordinaten der jeweiligen Einheit 2 sowie deren Geschwindigkeit hinsichtlich Betrag und Richtung.

Im dritten Schritt 14 des zur Realisierung des Signalpfades 41 vorgesehenen Verfahrensstrangs wird mittels der Steuerungseinrichtung 9 überprüft, ob eine Abweichung zwischen einer tatsächlich erfolgenden Bewegung der Einheit 2, die anhand der Bewegungssignale 25 bestimmt wird, und einer Bewegung der Einheit 2, die anhand der Steuersignale 20 zu erwarten ist, vorhanden ist. Hierbei erfolgt konkret ein Abgleich der ersten Bewegungsinformation 27 und der zweiten Bewegungsinformation 28 hinsichtlich einer vorliegenden Übereinstimmung. Denkbar ist diesbezüglich, dass von einer Fehlersituation ausgegangen wird, wenn die Abweichungen der Koordinaten und/oder der Geschwindigkeit größer sind als ein jeweiliger, insbesondere fest vorgegebener, Grenzwert. Ist dies nicht der Fall, dann wird das Verfahren erneut im ersten Schritt 12 des zur Realisierung des Signalpfades 41 vorgesehenen Verfahrensstrangs mit der Generierung der Steuersignale 20 und der anschließenden Erfassung der Bewegungssignale 25 fortgesetzt.

Sofern die im Schritt 14 erfolgende Überprüfung ergeben hat, dass diesbezüglich eine Abweichung vorhanden ist, dann werden im vierten Schritt 15 des zur Realisierung des Signalpfades 41 vorgesehenen Verfahrensstrangs mittels der Steuerungseinrichtung 9 Fehlersignale 29 generiert. Obgleich das Vorhandensein einer Abweichung grundsätzlich auf einen Fehler seitens der Bewegungserfassungseinrichtung 26 zurückzuführen sein könnte, besteht auch die Möglichkeit, dass die aktuell vorliegende Bewegung nicht dem entspricht, das im Zuge des aktuellen Betriebs der medizinischen Anlage 1 und gemäß der Durchführungsvorschrift respektive den Nutzersignalen erfolgen sollte. Potentiell liegt könnte in diesem Fall eine Gefahr von der sich bewegenden Einheit 2 ausgehen. Die Fehlersignale 29 sind auf ein Reduzieren oder ein Beseitigen dieser Gefahr gerichtet.

Die Fehlersignale 29 bewirken einerseits ein Anhalten der Bewegung der Einheit 2. So stellen die Fehlersignale 29 Steuersignale 20 dar, die ein Anhalten der Bewegung der Einheit 2 zur Folge haben. Zudem bewirken die Fehlersignale 29 die Ausgabe einer Fehlermitteilung 30 über eine als ein Touchscreen ausgebildete Ausgabeeinrichtung 31 der medizinischen Anlage 1. Hierbei wird dem Nutzer 21 ein Warntext angezeigt oder eine akustische Warnausgabe ausgegeben, der oder die diesen auf eine möglicherweise aktuell vorliegende Fehlfunktion bezüglich der Bewegung hinweist.

Parallel zu den soeben erläuterten Schritten 12 bis 15 erfolgt im ersten Schritt 16 des zur Realisierung des Sicherheitspfades 42 vorgesehenen Verfahrensstrangs die Erfassung dreidimensionaler Bilddaten 32, die über eine Übertragungsleitung des Sicherheitspfades 42 zur Überwachungseinrichtung 10 übertragen werden. Die Bilddaten 32 betreffend den Bereich der medizinischen Anlage 1 und zeigen mithin mitunter die Einheit 2. Zur Erfassung der Bilddaten 32 ist eine an der Decke 8 angeordnete Bilderfassungseinrichtung 33 vorgesehen, die mehrere 3D-Kameras 34 umfasst. Die Blickfelder der 3D-Kameras 34 sind unterschiedlich zueinander und nach unten gerichtet, sodass der komplette Bereich der medizinischen Anlage erfasst wird. Zumindest werden alle denkbaren Positionen der Einheiten 2 vom Blickfeld zumindest einer der 3D-Kameras 34 erfasst.

Die einer der 3D-Kameras 34 erfassten Bilddaten 32 liegen als ein zweidimensionales Array an Pixeln vor, wobei jedem Pixel einen Tiefenwert zugeordnet ist, der einen Abstand zur jeweiligen 3D-Kamera 34 zugeordnet ist. Die Auswertung der Bilddaten 32 erfolgt mittels der Überwachungseinrichtung 10, auf der eine entsprechend hierauf gerichtete Bildauswertungssoftware implementiert ist. Hierbei erfolgt mitunter eine Segmentierung der Bilddaten 32, bei der die in den Bilddaten 32 vorhandenen Bereiche den Einheiten 2 und weiteren Komponenten sowie Objekten zugeordnet werden. Anhand dieser Bilddaten 32 wird eine dritte Bewegungsinformation 35 mittels der Überwachungseinrichtung 10 ermittelt. Hierbei erfolgt die Auswertung der Bilddaten 32 hinsichtlich der Erkennung von Referenzpunkten der Einheit 2, die etwa an der Einheit 2 angebrachte Markierungen und/oder markante Stellen der Einheit 2, etwa Ecken oder Kanten, sein können. Hierbei werden auch bekannte Abmessungen und Geometrien der Einheit 2 genutzt. Die dritte Bewegungsinformation 35 umfasst die Koordinaten der jeweiligen Einheit 2 sowie deren Geschwindigkeit hinsichtlich Betrag und Richtung.

Im zweiten Schritt 17 des zur Realisierung des Sicherheitspfades 42 vorgesehenen Verfahrensstrangs werden mittels der Steuerungseinrichtung 9 Erwartungssignale 36 generiert und an die Überwachungseinrichtung 10 ausgegeben. Die Erwartungssignale 35 werden, gleichermaßen wie die Steuersignale 20, anhand der zu generierenden Bewegung der Einheit 2 generiert. Die Überwachungseinrichtung 10 ermittelt aus den Erwartungssignalen 36 eine vierte Bewegungsinformation 37, die die bei fehlerfreier Ausführung der Steuersignale 20 zu erwartenden Koordinaten der jeweiligen Einheit 2 sowie deren zu erwartende Geschwindigkeit angibt.

Im dritten Schritt 18 des zur Realisierung des Sicherheitspfades 42 vorgesehenen Verfahrensstrangs wird mittels der Überwachungseinrichtung 10 überprüft, ob eine Abweichung zwischen der tatsächlich erfolgenden Bewegung der jeweiligen Einheit 2, die anhand der Bilddaten 32 bestimmt wurde, und der Bewegung der jeweiligen Einheit 2, die anhand der Erwartungssignale 36 zu erwarten ist, vorhanden ist. Hierbei erfolgt konkret ein Abgleich der dritten Bewegungsinformation 35 und der vierten Bewegungsinformation 37 hinsichtlich einer vorliegenden Übereinstimmung. Denkbar ist diesbezüglich, dass von einer Fehlersituation ausgegangen wird, wenn die Abweichungen der Koordinaten und/oder der Geschwindigkeit größer sind als ein jeweiliger, insbesondere fest vorgegebener, Grenzwert. Ist dies nicht der Fall, dann wird das Verfahren erneut im ersten Schritt 16 des zur Realisierung des Sicherheitspfades 42 vorgesehenen Verfahrensstrangs mit der Erfassung der Bilddaten 32 und der anschließenden Erfassung der Erwartungssignale 36 fortgesetzt.

Sofern die Überprüfung im dritten Schritt 18 des zur Realisierung des Sicherheitspfades 42 vorgesehenen Verfahrensstrangs ergeben hat, dass diesbezüglich eine Abweichung vorhanden ist, dann werden im vierten Schritt 19 des zur Realisierung des Sicherheitspfades 42 vorgesehenen Verfahrensstrangs mittels der Überwachungseinrichtung 10 Fehlersignale 38 generiert. Obgleich das Vorhandensein einer Abweichung grundsätzlich auf einen Fehler seitens der Bilderfassungseinrichtung 33 zurückzuführen sein könnte, etwa da die Daten eines eingefrorenen Bildes geliefert werden, besteht die Möglichkeit, dass die aktuell vorliegende Bewegung nicht dem entspricht, dass im Zuge des aktuellen Betriebs der medizinischen Anlage 1 erfolgen soll. Potentiell liegt mithin auch in diesem Fall potentiell eine von der sich bewegenden Einheit 2 ausgehende Gefahrvor. Die mittels der Überwachungseinrichtung 10 generierten Fehlersignale 38 sind, gleichermaßen wie die mittels der Steuerungseinrichtung 9 generierten Fehlersignale 29, auf ein Reduzieren oder ein Beseitigen dieser Gefahr gerichtet.

So bewirken die Fehlersignale 38 ein Anhalten der Bewegung der Einheit 2. die Fehlersignale 38 werden hierzu an eine Unterbrechungseinrichtung 39 und eine Bremseinrichtung 40 ausgegeben. Seitens der Unterbrechungseinrichtung 39 bewirken die Fehlersignale 38 eine Unterbrechung der Zufuhr der Betriebsspannung zum jeweiligen Aktor 6. Die Unterbrechungseinrichtung 39 ist als ein durch die Fehlersignale 38 ansteuerbarer Schalter ausgebildet, der die Stromzufuhr zum jeweiligen Aktor 6 beim Vorliegen der entsprechenden Fehlersignale 38 unterbricht. Die Bremseinrichtung 40 weist Bremsvorrichtungen wie etwa Bremsscheiben und/oder -backen auf, die bei Vorliegen der Fehlersignale 38 ausgelöst werden. Hierbei werden auf die sich gegebenenfalls noch bewegenden Komponente der jeweiligen Einheit 2 respektive des jeweiligen Aktors 6 Bremskräfte generiert, sodass etwaige, aufgrund der Massenträgheit oder der Schwerkraft weiterhin vorliegende Bewegungen der Einheit 2 abgebremst und gestoppt werden. Zudem bewirken die Fehlersignale 38 die Ausgabe der bereits im Zusammenhang mit dem vierten Schritt 15 des zur Realisierung des Signalpfades 41 vorgesehenen Verfahrensstrangs erläuterten Fehlermitteilung 30 über die Ausgabeeinrichtung 31.

Die soeben erläuterten Schritte 12 bis 19 realisieren eine Erstfehlersicherheit bei der medizinischen Anlage 1 dadurch, indem mittels der Schritte 12 bis 15 ein Signalpfad 41 und mittels der Schritte 16 bis 19 ein Sicherheitspfad 42 realisiert wird. Die im Zuge der Realisierung der Pfade 41, 42 verwendeten Komponenten, insbesondere die Überwachungseinrichtung 10 und die Steuerungseinrichtung 9 sowie die zur Signalübertragung der jeweiligen Pfade 41, 42 vorgesehenen Übertragungsleitungen, sind als separate Komponenten ausgebildet, die unabhängig voneinander arbeiten, sodass sich ein in einem der Pfade 41, 42 vorliegender Fehler nicht auf den jeweils anderen Pfad 41, 42 fortpflanzt. Hinsichtlich der Fehlersignale 29, 38 wird durch die konkrete Realisierung der beiden Pfade 41, 42 eine Redundanz geschaffen.

Wie bereits angesprochen wurde, umfasst die medizinische Anlage 1 mehrere bewegliche Einheiten 2, nämlich den Patiententisch 3 sowie die Bildgebungseinheit 5. Die zuvor erläuterten Schritte 12 bis 19 werden für jede der Einheiten 2 separat durchgeführt, sodass die Bewegungsüberwachung bezüglich aller beweglichen Einheit 2 durchgeführt wird. Auch ist vorgesehen, dass die Bewegung der jeweiligen Einheit 2 mittels mehrerer Aktoren 6 bewerkstelligt wird, sodass die Bewegung der jeweiligen Einheit 2 eine Gesamtbewegung ist, die sich aus Einzelbewegungen zusammensetzt, die jeweils mittels einem der Aktoren 6 generiert werden. Hierdurch werden Multi-Achs-Bewegungen der Einheiten 2 ermöglicht. Die einzelnen Bewegungen sind hierbei lineare Bewegungen oder Schwenkbewegungen, die sich in Summe zu der Gesamtbewegung zusammensetzen und etwa eine gekrümmte Trajektorie für die jeweilige Einheit 2 ermöglichen. Im Rahmen dieser Ausführungsform wird im dritten Schritt 18 des zur Realisierung des Sicherheitspfades 42 vorgesehenen Verfahrensstrangs überprüft, ob eine Abweichung zwischen der tatsächlich erfolgenden Gesamtbewegung der jeweiligen Einheit 2, die anhand der Bilddaten 32 bestimmt wird, und der Gesamtbewegung der jeweiligen Einheit 2, die anhand der Erwartungssignale 36 zu erwarten ist, vorhanden ist.

Ferner sei adressiert, dass die Schritte 12 bis 15 und die Schritte 16 bis 19 zeitgleich und parallel zueinander durchgeführt werden, wobei, sofern die Überprüfung im dritten Schritt 14 des zur Realisierung des Signalpfades 41 vorgesehenen Verfahrensstrangs beziehungsweise im Schritt 18 ergibt, dass keine Abweichung vorhanden ist, jeder dieser beiden Verfahrensstränge erneut in dem für den jeweiligen Verfahrensstrang ersten Schritt 12, 16 begonnen werden. Die Schritte 12 bis 14 sowie 16 bis 18 werden mithin zyklischen durchlaufen, wobei laufend eine neue Erfassung und mithin Aktualisierung der Signale respektive Daten 20, 45, 32, 36 erfolgt. Die Zeitdauer für die Durchführung dieser Zyklen kann maximal 1 Sekunde betragen, beispielsweise eine Zehntel Sekunde.

Nachfolgend werden weitere, optionale Aspekte des Verfahrens hinsichtlich des vierten Schritts 15 des zur Realisierung des Signalpfades 41 vorgesehenen Verfahrensstrangs sowie des vierten Schritts 19 des zur Realisierung des Sicherheitspfades 42 vorgesehenen Verfahrensstrangs erläutert. So erfolgt in diesen Schritten 15, 19 jeweils die Generierung und Ausgabe von Fehlersignale 29, 38, die neben dem unmittelbaren Anhalten der Bewegung der jeweiligen Einheit 2 die zusätzliche Ausgabe der Fehlermitteilung 30 an den Nutzer 21 bewirken. Denkbar ist in dieser Situation, dass ein Techniker konsultiert wird, der überprüft, bezüglich welcher der Komponente der medizinischen Anlage 1 der Fehler vorliegt und diesen gegebenenfalls beseitigt. Diesem Fall kann der jeweilige Verfahrensstrang erneut von vorne begonnen werden. Denkbar ist aber auch, dass bereits der Nutzer 21 erkennt, dass aktuell bezüglich der Bewegung der Einheit 2 in Realität keine Fehlersituation respektive keine Gefahr vorliegt, sodass, etwa um zusätzliche Belastungen für den Patienten 4 zu vermeiden, eine Fortsetzung des Betriebs der medizinischen Anlage 1 respektive der Bewegung der jeweiligen Einheit 2 zweckmäßig ist. Hierauf sind die nachfolgend noch erläuterten Schritte 43, 44, die sich optional an die Schritte 15, 19 anschließen, gerichtet. In dem in der Fig. 3 gezeigten Flussdiagramm sind diese optionalen Schritte 43, 44 gestrichelt angedeutet.

In dem jeweiligen Schritt 43, 44 erfolgt die Überprüfung einer Freigabebedingung, wobei hierzu bezüglich des Schritts 43 die Steuerungseinrichtung 9 und bezüglich des Schritts 44 die Überwachungseinrichtung 10 eingerichtet und vorgesehen ist. So wird dem Nutzer 21 zusätzlich zu der Fehlermitteilung 30 ein Button mittels der Ausgabeeinrichtung 31 angezeigt, auf dem der Text "Vorgang dennoch fortsetzen" angezeigt ist. Der Nutzer 21 kann den Button mittels Anklicken oder Antippen auswählen. Die entsprechende Auswahl dieses Buttons bewirkt die Generierung eines Freigabesignals 45, bei dessen Vorliegen die Freigabebedingung erfüllt ist. Die Ausgabeeinrichtung 31 fungiert mithin zudem als eine Eingabevorrichtung 53 der medizinischen Anlage 1. Das Freigabesignal 45, das an die Steuerungseinrichtung 9 beziehungsweise die Überwachungseinrichtung 10 übertragen wird, bewirkt ein Fortsetzen des Verfahrens im Rahmen des ersten Schritts 12 des zur Realisierung des Signalpfades 41 vorgesehenen Verfahrensstrangs respektive des ersten Schritts 16 des zur Realisierung des Sicherheitspfades 42 vorgesehenen Verfahrensstrangs. Folglich bewirkt die Erfüllung der Freigabebedingung ein Fortsetzen der Bewegung der jeweiligen Einheit 2, vorliegend beispielhaft jedoch nur mit reduzierter Geschwindigkeit. Denkbar ist auch, dass die Schritte 12 bis 15 beziehungsweise 16 bis 19 des jeweils fehlerbehafteten Pfades 41, 42 bei erfüllter Freigabebedingung nicht mehr durchgeführt respektive unterdrückt werden.

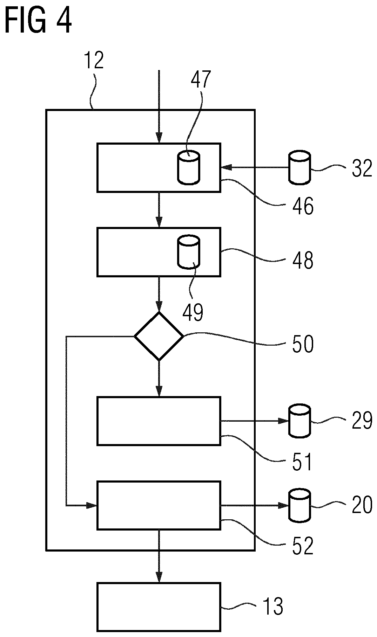

Nachfolgend wird unter Bezugnahme auf die Fig. 4 ein denkbarer Aspekt bezüglich des ersten Schritts 12 des zur Realisierung des Signalpfades 41 vorgesehenen Verfahrensstrangs erläutert. So zeigt Fig. 4 weitere Details bezüglich des Schritts 12 als Flussdiagramm. So werden in einem Schritt 46 die Bilddaten 32 an die Steuerungseinrichtung 9 übertragen, die gemäß dem nachfolgend Erläuterten zur Generierung der Steuersignale 32 genutzt werden. So ermittelt die Steuerungseinrichtung 9 anhand der Bilddaten 32 eine Einheitinformation 47, die die aktuelle Bewegung, also die aktuelle Position sowie die aktuelle Geschwindigkeit, der jeweiligen Einheit 2 betreffen, wobei diesbezüglich dasselbe Vorgehen wie zur Ermittlung der dritten Bewegungsinformation 35 mittels der Überwachungseinrichtung 10 vorgesehen ist.

Im darauffolgenden Schritt 48 wird eine Objektinformation 49 anhand der Bilddaten 32 ermittelt, die eine aktuelle Position und/oder Bewegung eines sich im Bereich der medizinischen Anlage 1 befindenden Objekts betrifft, das ebenfalls in den Bilddaten 32 abgebildet wird. Das Objekt kann im Bereich der medizinischen Anlage 1 ortsfest sein oder sich bewegen. Auch hier erfolgt diesbezüglich dasselbe Vorgehen respektive eine analoge Auswertung der Bilddaten 32 hinsichtlich des Objekts wie zur Ermittlung der Bewegungsinformation 35 respektive der Einheitinformation 47.

Im nächsten Schritt 50 erfolgt die Überprüfung der Erfüllung einer Kollisionsbedingung, die dann erfüllt ist, wenn sich anhand der Einheitinformation 47 sowie der Objektinformation 49 ergibt, dass aktuell die Gefahr einer Kollision der jeweiligen Einheit 2 mit dem jeweiligen Objekt gegeben ist. Hierzu werden unter Nutzung der Einheitinformation 47 und der Objektinformation 49 voraussichtliche Trajektorien der jeweiligen Einheit 2 und gegebenenfalls des Objekts ermittelt, wobei die Kollisionsbedingung dann erfüllt ist, wenn diese Trajektorien einen Schnittpunkt oder, allgemein gesprochen, einen gemeinsamen Bereich aufweisen. Hierbei werden auch bekannte Abmessungen und Geometrien der Einheit 2 sowie gegebenenfalls des Objekts genutzt. Entsprechend liegt die Trajektorie als der Volumenbereich des Bereichs der medizinischen Anlage 1 vor, die von der Einheit 2 respektive dem Objekt überstrichen wird. Sofern das Objekt ortsfest ist, liegt diesbezüglich keine Trajektorie, sondern stattdessen der entsprechende Volumenbereich des Bereichs der medizinischen Anlage 1 vor, in dem sich das Objekt befindet respektive der von dem Objekt ausgefüllt wird. Die Kollisionsbedingung ist dann erfüllt, wenn diese Volumenbereiche nicht disjunkt sind. Zusätzlich wird bezüglich der Überprüfung der Erfüllung der Kollisionsbedingung eine zeitliche Bedingung überprüft, die dann erfüllt ist, wenn sich anhand der voraussichtlichen Trajektorien ergibt, dass sich die jeweilige Einheit 2 und das Objekt zum selben Zeitpunkt an derselben Position respektive dem Schnittpunkt befinden werden.

Falls die Kollisionsbedingung erfüllt ist, dann werden die Steuersignale 20 derart generiert, dass die Gefahr bezüglich dieser Kollision verringert oder beseitigt wird. Entsprechend kann in diesem Fall gemäß dem oben anhand dem vierten Schritt 15 des zur Realisierung des Signalpfades 41 vorgesehenen Verfahrensstrangs Erläuterten vorgegangen werden, sodass im nächsten Schritt 51 entsprechende Fehlersignale 29 generiert und ausgegeben werden. Konkret kann in diesem Fall auch ein Vorgehen gemäß dem in der europäischen Patentanmeldung mit dem Aktenzeichen EP 23200297.2 Beschriebenen vorgesehen sein. Bei der Nichterfüllung der Kollisionsbedingung werden im Rahmen des nächsten Schritt 52 die Steuersignale 20 anhand der Durchführungsvorschrift und/oder der Nutzersignale generiert und an den Aktor 6 respektive den Motor-Controller 23 ausgegeben.

Unabhängig vom grammatikalischen Geschlecht eines bestimmten Begriffes sind Personen mit männlicher, weiblicher oder anderer Geschlechteridentität mit umfasst.

## Patentansprüche

1. Verfahren zur Überwachung einer Bewegung wenigstens einer bewegbaren Einheit (2) einer medizinischen Anlage (1), wobei das Verfahren die folgenden Schritte umfasst:
(i) Generieren der Bewegung der Einheit (2) mittels wenigstens eines Aktors (6) dadurch, dass mittels einer Steuerungseinrichtung (9) auf diese Bewegung gerichtete Steuersignale (20) generiert und über eine Übertragungsleitung eines Signalpfades (41) an den Aktor (6) oder eine zur Ansteuerung des Aktors (6) vorgesehene Komponente übertragen werden,
(ii) Generieren von Bewegungssignalen (25) anhand einer tatsächlich erfolgenden Bewegung der Einheit (2) mittels einer Bewegungserfassungseinrichtung (26) und Übertragen der Bewegungssignale (25) an die Steuerungseinrichtung (9) über die oder eine weitere Übertragungsleitung des Signalpfades (41),
(iii) Überprüfen mittels der Steuerungseinrichtung (9), ob eine Abweichung zwischen einer tatsächlich erfolgenden Bewegung der Einheit (2), die anhand der Bewegungssignale (25) bestimmt wird, und einer Bewegung der Einheit (2), die anhand der Steuersignale (20) zu erwarten ist, vorhanden ist,
(iv) Generieren von Fehlersignalen (29) mittels der Steuerungseinrichtung (9), falls die im Schritt (iii) erfolgende Überprüfung das Vorhandensein einer solchen Abweichung ergeben hat, wobei die Fehlersignale (29) auf ein Reduzieren oder Beseitigen einer von der Bewegung der Einheit (2) potentiell ausgehenden Gefahr gerichtet sind,
(v) Erfassen dreidimensionaler und die Einheit (2) betreffender Bilddaten (32) mittels einer Bilderfassungseinrichtung (33), wobei die Bilddaten (32) über eine Übertragungsleitung eines Sicherheitspfades (42) an eine Überwachungseinrichtung (10) übertragen werden,
(vi) Generieren von Erwartungssignalen (36) mittels der Steuerungseinrichtung (9), wobei die Erwartungssignale die zu generierende Bewegung der Einheit (2) betreffen, und Übertragen der Erwartungssignale an die Überwachungseinrichtung (10),
(vii) Überprüfen mittels der Überwachungseinrichtung (10), ob eine Abweichung zwischen einer tatsächlich erfolgenden Bewegung der Einheit (2), die anhand der Bilddaten (32) bestimmt wird, und einer Bewegung der Einheit (2), die anhand der Erwartungssignale (36) zu erwarten ist, vorhanden ist,
(viii) Generieren von Fehlersignalen (38) mittels der Überwachungseinrichtung (10), falls die im Schritt (vii) erfolgende Überprüfung das Vorhandensein einer solchen Abweichung ergeben hat, wobei die Fehlersignale (38) auf ein Reduzieren oder Beseitigen einer von der Bewegung der Einheit (2) potentiell ausgehenden Gefahr gerichtet sind.

2. Verfahren nach Anspruch 1, **dadurch gekennzeichnet, dass** wenigstens ein einen Frequenzumrichter realisierender Motor-Controller (23) vorgesehen ist, mittels dem der hiermit verbundene elektromechanische Aktor (6) mit einer zum Generieren der Bewegung der Einheit (2) erforderlichen Betriebsspannung beaufschlagbar ist, wobei die in Schritt (i) generierten Steuersignale (20) zur Steuerung des Aktors (6) zum Motor-Controller (23) übertragen werden.

3. Verfahren nach Anspruch 1 oder 2, **dadurch gekennzeichnet, dass** die Bewegungserfassungseinrichtung (26) eine Kodiereinrichtung ist, mittels der die tatsächlich erfolgende Bewegung der Einheit (2) und/oder des Aktors (6) im Rahmen des Schritts (ii) in die Bewegungssignale (25) umgewandelt wird.

4. Verfahren nach einem der vorangehenden Ansprüche, **dadurch gekennzeichnet, dass** die Bewegung der Einheit (2) mittels mehrerer Aktoren (6) durch die Generierung der Steuersignale (20), die an die Aktoren (6) oder die zur Ansteuerung der Aktoren (6) vorgesehenen Komponenten übertragen werden, generiert wird, sodass die Bewegung eine Gesamtbewegung ist, die sich aus Einzelbewegungen, die mittels der Aktoren (6) jeweils generiert werden, zusammensetzt, wobei in Schritt (vii) überprüft wird, ob eine Abweichung zwischen der tatsächlich erfolgenden Gesamtbewegung der Einheit (2), die anhand der Bilddaten (32) bestimmt wird, und der Gesamtbewegung der Einheit (2), die anhand der Erwartungssignale (36) zu erwarten ist, vorhanden ist.

5. Verfahren nach einem der vorangehenden Ansprüche, **dadurch gekennzeichnet, dass** die im Rahmen des Schritts (iv) oder des Schritts (viii) generierten Fehlersignale (29, 38) ein Anhalten der Bewegung der Einheit (2) bewirken, wobei
- die im Rahmen des Schritts (iv) generierten Fehlersignale (29) seitens der Steuerungseinrichtung (9) generierte Steuersignale (20) sind, die ein Anhalten der Bewegung der Einheit (2) bewirken, und/oder
- die im Rahmen des Schritts (viii) generierten Fehlersignale (38) seitens der Überwachungseinrichtung (10) generiert und an eine Unterbrechungseinrichtung (39) und/oder eine Bremseinrichtung (40) ausgegeben werden und eine mittels der Unterbrechungseinrichtung (39) bewirkte Unterbrechung einer Beaufschlagung des Aktors (6) mit der oder einer Betriebsspannung und/oder ein Festsetzen der Einheit (2) mittels der Bremseinrichtung (40) bewirken.

6. Verfahren nach einem der vorangehenden Ansprüche, **dadurch gekennzeichnet, dass** die im Rahmen des Schritts (iv) oder des Schritts (viii) generierten Fehlersignale (29, 38) an eine Ausgabeeinrichtung (31) übertragen werden und die mittels der Ausgabeeinrichtung (31) erfolgende Ausgabe einer Fehlermitteilung (30) bewirken, mittels der ein Nutzer (21) auf das Vorliegen einer potentiellen Störsituation hingewiesen wird.

7. Verfahren nach Anspruch 5 und 6, **dadurch gekennzeichnet, dass** nach erfolgter Generierung der das Anhalten der Bewegung der Einheit (2) und die Ausgabe der Fehlermitteilung (30) bewirkenden Fehlersignale (29, 38) zusätzlich die Erfüllung einer Freigabebedingung überprüft wird, wobei die Freigabebedingung erfüllt ist, wenn ein mittels einer Eingabevorrichtung (53) generiertes Freigabesignal (45) vorliegt, wobei die Generierung des Freigabesignals (45) mittels einer nutzerseitig an der Eingabevorrichtung (53) vorgenommenen und auf die Freigabe der Bewegung gerichteten Nutzerhandlung erfolgt, wobei die Bewegung der Einheit (2) nur bei der Erfüllung der Freigabebedingung, insbesondere im Rahmen einer reduzierten Geschwindigkeit, fortgesetzt wird.

8. Verfahren nach einem der vorangehenden Ansprüche, **dadurch gekennzeichnet, dass** die Bilddaten (32) zur Steuerungseinrichtung (9) übertragen werden, wobei die Steuersignale (20) anhand der Bilddaten (32) generiert werden.

9. Verfahren nach Anspruch 8, **dadurch gekennzeichnet, dass** mittels der Steuerungseinrichtung (9) und anhand der Bilddaten (32) wenigstens eine eine aktuelle Position und/oder Bewegung der Einheit (2) betreffende Einheitinformation (47) und eine Objektinformation (49) betreffend eine aktuelle Position und/oder Bewegung eines sich im Bereich der medizinischen Anlage (1) befindenden Objekts ermittelt wird, wobei die Steuersignale (20) anhand der wenigstens einen Einheitininformation (47) und/oder der wenigstens einen Objektinformation (49) generiert werden, wobei mittels der Steuerungseinrichtung (9) die Erfüllung einer Kollisionsbedingung überprüft wird, die dann erfüllt ist, wenn sich anhand der wenigstens einen Einheitinformation (47) und der wenigstens einen Objektinformation (49) ergibt, dass aktuell die Gefahr einer Kollision der Einheit (2) und des Objekts gegeben ist, wobei mittels der Steuerungseinrichtung (9) bei der Erfüllung der Kollisionsbedingung die Steuersignale (20) derart generiert werden, dass die Gefahr verringert oder beseitigt wird.

10. Medizinische Anlage (1), umfassend wenigstens eine bewegbare Einheit (2) und eine Steuerungseinrichtung (9), die dazu eingerichtet ist, auf die Bewegung der Einheit (2) gerichtete und diese Bewegung bewirkende Steuersignale (20) zu generieren und über eine Übertragungsleitung eines Signalpfades (41) an einen Aktor (6) oder eine zur Ansteuerung des Aktors (6) vorgesehene Komponente zu übertragen, und eine Bewegungserfassungseinrichtung (26), mittels der anhand einer tatsächlich erfolgenden Bewegung der Einheit (2) Bewegungssignale (25) generierbar und an die Steuerungseinrichtung (9) über die oder eine weitere Übertragungsleitung des Signalpfades (41) übertragbar sind, wobei die Steuerungseinrichtung (9) ferner dazu eingerichtet ist, zu überprüfen, ob eine Abweichung zwischen einer tatsächlich erfolgenden Bewegung der Einheit (2), die anhand der Bewegungssignale (25) bestimmbar ist, und einer Bewegung der Einheit (2), die anhand der Steuersignale (20) zu erwarten ist, vorhanden ist, und im Falle des Vorhandenseins einer solchen Abweichung Fehlersignale (29) zu generieren, wobei die Fehlersignale (29) auf ein Reduzieren oder Beseitigen einer von der Bewegung der Einheit (2) potentiell ausgehenden Gefahr gerichtet sind, wobei die medizinische Anlage (1) weiterhin eine Bilderfassungseinrichtung (33) umfasst, mittels der dreidimensionale und die Einheit (2) betreffende Bilddaten (32) erfassbar und über eine Übertragungsleitung eines Sicherheitspfades (42) an eine Überwachungseinrichtung (10) der medizinischen Anlage (1) übertragbar sind, wobei die Steuerungseinrichtung (9) ferner dazu eingerichtet ist, Erwartungssignale (36), die die zu generierende Bewegung der Einheit (2) betreffen zu generieren und an die Überwachungseinrichtung (10) auszugeben, wobei die Überwachungseinrichtung (10) dazu eingerichtet ist, zu überprüfen, ob eine Abweichung zwischen einer tatsächlich erfolgenden Bewegung der Einheit (2), die anhand der Bilddaten (32) bestimmbar ist, und einer Bewegung der Einheit (2), die anhand der Erwartungssignale (36) zu erwarten ist, vorhanden ist, und im Falle des Vorhandenseins einer solchen Abweichung Fehlersignale (38) zu generieren, wobei die Fehlersignale (38) auf ein Reduzieren oder Beseitigen einer von der Bewegung der Einheit (2) potentiell ausgehenden Gefahr gerichtet sind.

11. Medizinische Anlage (1) nach Anspruch 10, **dadurch gekennzeichnet, dass** diese eine medizinische Bildgebungseinrichtung, insbesondere eine Magnetresonanztomographie-Einrichtung oder eine Computertomographie-Einrichtung oder ein Angiographiegerät oder ein Ultraschallgerät oder eine Positronen-Emissions-Tomographie-Anlage, oder eine Behandlungseinrichtung, insbesondere eine Strahlentherapie-Anlage, ist.

12. Medizinische Anlage (1) nach Anspruch 11, **dadurch gekennzeichnet, dass** die Einheit (2) oder eine der Einheiten (2) ein Patientenlagerungstisch (3) zur Aufnahme eines Patienten (4) zur Durchführung einer Bildgebung oder Behandlung oder eine Bildgebungseinheit (5), insbesondere ein C-Bogen oder eine Vorrichtung zur Erzeugung einer ionisierenden Strahlung, ist.

13. Steuergerät (11) für eine medizinische Anlage (1) nach einem der Ansprüche 10 bis 12, **dadurch gekennzeichnet, dass** das Steuergerät (11) als eine Steuerungseinrichtung (9) oder eine Überwachungseinrichtung (10) ausgebildet ist, die zur Durchführung des Verfahrens nach einem der Ansprüche 1 bis 9 eingerichtet ist.

14. Computerprogramm, welches in eine Speichereinheit eines Steuergeräts (11) nach Anspruch 13 ladbar ist, umfassend Programmabschnitte, um Schritte des Verfahrens nach einem der Ansprüche 1 bis 9 auszuführen, wenn das Computerprogramm seitens des Steuergeräts (11) ausgeführt wird.

15. Computerlesbares Medium, auf welchem von einem Steuergerät (11) nach Anspruch 13 einlesbare und ausführbare Programmabschnitte gespeichert sind, um Schritte des Verfahrens nach einem der Ansprüche 1 bis 9 auszuführen, wenn die Programmabschnitte seitens des Steuergeräts (11) ausgeführt werden.
